(19) Europäisches Patentamt
European Patent Office
Office européen des brevets

(11) Veröffentlichungsnummer: **0 212 107 A1**

(12) **EUROPÄISCHE PATENTANMELDUNG**

(21) Anmeldenummer: **86107778.2**

(22) Anmeldetag: **07.06.86**

(51) Int. Cl.[4]: **C 07 D 211/90**, C 07 D 401/04, C 07 C 143/72, A 61 K 31/44

(30) Priorität: **21.06.85 CH 2651/85**

(43) Veröffentlichungstag der Anmeldung: **04.03.87 Patentblatt 87/10**

(84) Benannte Vertragsstaaten: **BE CH DE FR GB IT LI NL SE**

(71) Anmelder: **F. HOFFMANN-LA ROCHE & CO. Aktiengesellschaft, CH-4002 Basel (CH)**

(72) Erfinder: **Jaunin, Roland, Prof. Dr., Lehenmattstrasse 308, CH-4052 Basel (CH)**
Erfinder: **Ramuz, Henri, Prof. Dr., Rheinparkstrasse 3, CH-4127 Birsfelden (CH)**

(74) Vertreter: **Kellenberger, Marcus, Dr. et al, Grenzacherstrasse 124 Postfach 3255, CH-4002 Basel (CH)**

(54) **1,4-Dihydro-5-sulfamoyl-nicotinsäureester.**

(57) Es wurde gefunden, daß die neuen Dihydropyridinderivate der Formel

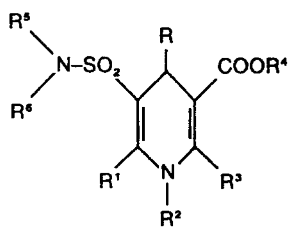

I

worin die Symbole R und $R^1$ bis $R^6$ die in Anspruch 1 angegebene Bedeutung haben, eine ausgeprägte Calcium-antagonistische Wirkung besitzen und demnach als Arzneimittel verwendet werden können, insbesondere bei der Bekämpfung bzw. Verhütung von Angina pectoris, Ischämie, Bluthochdruck und/oder Migräne. Die Verbindungen der Formel I können durch Umsetzen einer ebenfalls neuen Ylidenverbindung der Formel

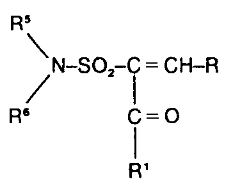

II

mit einem Enamin der Formel

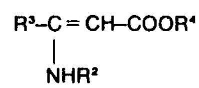

III

hergestellt werden.

EP 0 212 107 A1

F. Hoffmann-La Roche & Co. Aktiengesellschaft, Basel, Schweiz

RAN 4029/3

BEZEICHNUNG GEÄNDERT
siehe Titelseite

# 1,4-Dihydro-5-sulfamoyl-4-nicotinsäureester

Die vorliegende Erfindung betrifft Dihydropyridinderivate. Im Speziellen betrifft sie Dihydropyridinderivate der allgemeinen Formel

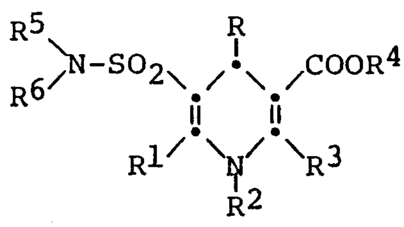

I

worin R Aryl oder einen heterocyclischen Rest mit bis zu drei Heteroatomen, wobei als Heteroatome Sauerstoff, Stickstoff oder Schwefel in Frage kommen, $R^1$ und $R^3$ je $C_1$-$C_4$-Alkyl oder $C_3$-$C_6$-Cycloalkyl, $R^2$ Wasserstoff oder $C_1$-$C_4$-Alkyl, $R^4$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_6$-Alkoxycarbonyl-$C_2$-$C_6$-alkyl, $C_1$-$C_4$-Alkanoyl-

oxy-$C_1$-$C_6$-alkyl, $C_3$-$C_6$-Alkenyloxy-$C_1$-$C_6$-alkyl, $C_1$-$C_6$-Alkylthio-$C_1$-$C_6$-alkyl, Benzyloxy-$C_1$-$C_6$-alkyl, gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl oder gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl-$C_1$-$C_6$-alkyl, $R^5$ Wasserstoff, $C_1$-$C_6$-Alkyl oder $C_3$-$C_6$-Cycloalkyl, $R^6$ $C_1$-$C_6$-Alkyl, $C_3$-$C_6$-Cycloalkyl, $C_3$-$C_6$-Cycloalkyl-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_6$-Alkylthio-$C_1$-$C_6$-alkyl, $C_1$-$C_6$-Alkoxycarbonyl-$C_2$-$C_6$-alkyl oder $R^5$ und $R^6$ zusammen die Gruppe $-CH_2CH_2-O-CH_2CH_2-$ oder eine $-(CH_2)_n$-Gruppe, worin n eine Zahl von 2 bis 7 bedeutet, bedeuten, in Form von Isomeren, Isomerengemischen, Racematen und optischen Antipoden.

Diese Verbindungen sind neu und zeichnen sich durch wertvolle pharmakodynamische Eigenschaften aus.

Gegenstand der vorliegenden Erfindung sind Verbindungen der allgemeinen Formel I als solche und zur Anwendung als therapeutische Wirkstoffe, die Herstellung dieser Verbindungen, Zwischenprodukte für die Herstellung dieser Verbindungen, ferner diese enthaltende Arzneimittel und die Herstellung solcher Arzneimittel, sowie die Verwendung von Verbindungen der allgemeinen Formel I bei der Bekämpfung bzw. Verhütung von Krankheiten bzw. bei der Verbesserung der Gesundheit, insbesondere bei der Bekämpfung bzw. Verhütung von Angina pectoris, Ischämie, Bluthochdruck und Migräne.

Der in der vorliegenden Beschreibung verwendete Ausdruck "Alkyl" - allein oder in Kombination - bedeutet geradkettige und verzweigte, gesättigte Kohlenwasserstoffreste mit der jeweils angegebenen Anzahl Kohlenstoffatome, wie Methyl, Aethyl, n-Propyl, Isopropyl, n-Butyl, Isobutyl, sec.-Butyl, tert.-Butyl und dergleichen. Der Ausdruck "Alkoxy" bedeutet Alkyläthergruppen, worin der Ausdruck "Alkyl" die obige Bedeutung hat. Der Ausdruck "$C_3$-$C_6$--Alkenyl" umfasst geradkettige und verzweigte Kohlenwasserstoffgruppen mit 3-6 Kohlenstoffatomen, worin mindestens eine Kohlenstoff-Kohlenstoff-Bindung ungesättigt ist, wie Allyl, Butenyl und dergleichen. Der Ausdruck "$C_3$-$C_6$--Alkenyloxy" bedeutet Alkenyläthergruppen, worin der Ausdruck "$C_3$-$C_6$-Alkenyl" die obige Bedeutung hat. Der Ausdruck "$C_3$-$C_6$-Alkinyl" bezieht sich auf geradkettige und verzweigte Kohlenwasserstoffgruppen mit 3-6 Kohlenstoffatomen, worin mindestens eine Kohlenstoff-Kohlenstoff-Dreifachbindung vorhanden ist, wie Propargyl und dergleichen. Der Ausdruck "$C_3$-$C_6$-Cycloalkyl" bezieht sich auf cyclische, gesättigte Kohlenwasserstoffreste mit 3-6 Kohlenstoffatomen, wie Cyclopropyl, Cyclohexyl und dergleichen. Der Ausdruck "$C_1$-$C_4$-Alkanoyloxy" bezieht sich auf den Acyloxyrest einer Alkancarbonsäure mit 1-4 Kohlenstoffatomen, wie Formyloxy, Acetoxy, Propionyloxy, Butyryloxy und dergleichen. Der Ausdruck "Halogen" umfasst die vier Halogenatome Fluor, Chlor, Brom und Jod. Der Ausdruck "Aryl" umfasst einen gegebenenfalls durch Phenyl, $C_1$-$C_6$-Alkyl, $C_3$-$C_6$-Alkenyl, $C_3$-$C_6$-Alkinyl, $C_1$-$C_6$-Alkoxy, Halogen, Trifluormethyl, Trifluormethoxy, Difluormethoxy, Trifluormethylthio, Difluormethylthio, Nitro, Cyan, Azido, $C_1$-$C_6$-Alkoxycarbonyl, Aminocarbonyl, Aminosulfonyl, $C_1$-$C_6$-Alkylthio, $C_1$-$C_6$-Alkylsulfonyl oder $C_1$--$C_6$-Alkanoyl mono-, di- oder tri-substituierten oder durch $C_3$-$C_5$-Alkylen oder Dioxy-$C_1$-$C_2$-alkylen disubstituierten mono- oder bicyclischen aromatischen Kohlenwasserstoffrest mit bis zu 10 Kohlenstoffatomen im aromatischen Ringgerüst, wie Chlorphenyl, Tolyl, $\alpha,\alpha,\alpha$-Trifluortolyl, Dichlorphenyl, Chlornitrophenyl, Naphthyl und dergleichen. Der Ausdruck "heterocyclischer

Rest" umfasst 5- und 6-gliedrige mono- und bicyclische, gegebenenfalls durch Phenyl, $C_1$-$C_6$-Alkyl, $C_3$-$C_6$-Alkenyl, $C_3$-$C_6$-Alkinyl, $C_1$-$C_6$-Alkoxy, Halogen, Trifluormethyl, Trifluormethoxy, Difluormethoxy, Trifluormethylthio, Difluormethylthio, Nitro, Cyan, Azido, $C_1$-$C_6$-Alkoxycarbonyl, Aminocarbonyl, Aminosulfonyl, $C_1$-$C_6$-Alkylthio, $C_1$-$C_6$-Alkylsulfonyl oder $C_1$-$C_6$-Alkanoyl mono-, di- oder tri-substituierte oder durch $C_3$-$C_5$-Alkylen oder Dioxy-$C_1$-$C_2$-alkylen disubstituierte Heterocyclen, wie Thienyl, Furyl, Pyrryl, Pyrazolyl, Imidazolyl, Oxazolyl, Isoxazolyl, Thiazolyl, Isothiazolyl, Pyridyl, N-Oxydopyridyl, Pyridazinyl, Pyrimidyl, Pyrazinyl, Chinolyl, Isochinolyl, Indolyl, Benzimidazolyl, Chinazolyl, Chinoxalyl, (2,1,3-Benzoxadiazol)-4-yl, (2,1,3-Benzothiadiazol)-4-yl, Benzofuranyl, Benzothienyl, Benzoxazolyl, Benzisoxazolyl, Benzothiazolyl, Benzisothiazolyl, Indazolyl und dergleichen. Der Ausdruck "$C_1$-$C_6$-Alkanoyl" bedeutet den Acylrest einer Alkancarbonsäure mit 1-6 Kohlenstoffatomen, wie Formyl, Acetyl, Propionyl, Butyryl und dergleichen.

Eine besondere Klasse von Verbindungen der Formel I sind solche, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, $R^5$ $C_1$-$C_6$-Alkyl, $R^6$ $C_1$-$C_6$-Alkyl, $C_1$-$C_6$-Alkoxy-$C_1$-$C_6$-alkyl oder $C_1$-$C_6$-Alkoxycarbonyl-$C_2$-$C_6$-alkyl oder $R^5$ und $R^6$ zusammen eine $-(CH_2)_n$-Gruppe, worin n eine Zahl von 2 bis 7 bedeutet, und R Naphthyl, gegebenenfalls durch $C_1$-$C_6$-Alkyl, Halogen, Trifluormethyl oder Nitro monosubstituiertes oder durch Halogen bzw. Halogen und Nitro disubstituiertes Phenyl, Imidazolyl oder Pyridyl bedeuten.

Eine bevorzugte Klasse von Verbindungen der Formel I

sind solche, worin R Aryl, vorzugsweise durch $C_1$-$C_6$-Alkyl, Halogen, Trifluormethyl oder Nitro substituiertes Phenyl, bedeutet. Ganz besonders bevorzugt bedeutet R 3-Nitrophenyl, 2-Chlor-5-nitrophenyl oder 2,5-Dichlorphenyl. $R^1$ bedeutet vorzugsweise Methyl. Die bevorzugte Bedeutung von $R^2$ ist Wasserstoff. $R^3$ bedeutet vorzugsweise $C_1$-$C_4$-Alkyl, besonders bevorzugt Methyl. Weitere bevorzugte Verbindungen der Formel I sind solche, worin $R^4$ $C_1$-$C_6$-Alkyl, Cyan-$C_2$-$C_6$-alkyl, Halogen-$C_2$--$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$--$C_6$-alkyl oder gegebenenfalls durch Halogen substituiertes Phenyl-$C_1$-$C_6$-alkyl, vorzugsweise $C_1$-$C_6$-Alkyl, ω,ω,ω-Trifluor-$C_1$-$C_6$-alkyl, $C_1$-$C_6$-Alkoxy--$C_1$-$C_6$-alkyl oder Phenyl-$C_1$-$C_6$-alkyl, ganz besonders bevorzugt Methyl, Isopropyl, 2,2,2-Trifluoräthyl, 2-Propoxyäthyl oder 1-Phenyläthyl, bedeutet. $R^5$ bedeutet vorzugsweise $C_1$-$C_6$-Alkyl, besonders bevorzugt Methyl, Aethyl oder Isopropyl. Die bevorzugte Bedeutung von $R^6$ ist $C_1$-$C_6$-Alkyl oder $C_1$-$C_6$-Alkoxy-$C_1$-$C_6$-alkyl, besonders bevorzugt Methyl, Aethyl, Isopropyl, Butyl oder Methoxyäthyl.

Aus dem obigen folgt, dass diejenigen Verbindungen der Formel I ganz besonders bevorzugt sind, worin R 3-Nitrophenyl, 2-Chlor-5-nitrophenyl oder 2,5-Dichlorphenyl, $R^1$ und $R^3$ je Methyl, $R^2$ Wasserstoff, $R^4$ Methyl, Isopropyl, 2,2,2-Trifluoräthyl, 2-Propoxyäthyl oder 1-Phenyläthyl, $R^5$ Methyl, Aethyl oder Isopropyl und $R^6$ Methyl, Aethyl, Isopropyl, Butyl oder Methoxyäthyl bedeuten.

Die bevorzugteste Gruppe von Verbindungen der Formel I sind diejenigen, worin R 3-Nitrophenyl, $R^1$ und $R^3$ je Methyl, $R^2$ Wasserstoff, $R^4$ Isopropyl oder 2-Propoxyäthyl, $R^5$ Methyl und $R^6$ Methyl, Butyl oder Methoxyäthyl

bedeuten.

Die bevorzugtesten Verbindungen der Formel I sind:

5-(Dimethylsulfamoyl)-1,4-dihydro-2,6-dimethyl-4-(3-nitrophenyl)nicotinsäure-2-propoxyäthylester,

5-(Butylmethylsulfamoyl)-1,4-dihydro-2,6-dimethyl-4-(3-nitrophenyl)nicotinsäureisopropylester und

1,4-Dihydro-2,6-dimethyl-5-[(2-methoxyäthyl)methylsulfamoyl]-4-(3-nitrophenyl)nicotinsäureisopropylester.

Die Verbindungen der obigen Formel I können erfindungsgemäss hergestellt werden, indem man eine Ylidenverbindung der allgemeinen Formel

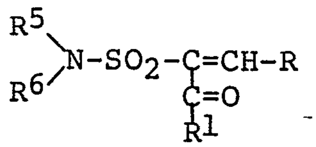

II

worin R, $R^1$, $R^5$ und $R^6$ die oben angegebene Bedeutung besitzen,

mit einem Enamin der allgemeinen Formel

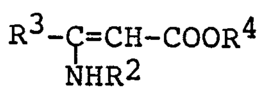

III

worin $R^2$, $R^3$ und $R^4$ die oben angegebene Bedeutung besitzen,

umsetzt, und erwünschtenfalls, ein erhaltenes Isomerengemisch in die Isomeren auftrennt.

Die Umsetzung einer Ylidenverbindung der Formel II mit einem Enamin der Formel III erfolgt nach an sich bekannten Methoden in Gegenwart einer Säure, wie p-Toluolsulfonsäure oder DL-Campher-10-sulfonsäure, oder der $H^+$-Form eines Kationenaustauschers, wie beispielsweise Amberlyst® 15, in einem inerten Lösungsmittel oder Lösungsmittelgemisch

bei einer Temperatur zwischen etwa 20° und 150°C, vorzugsweise bei der Rückflusstemperatur des Lösungsmittels oder Lösungsmittelgemisches. Für diesen Zweck geeignete Lösungsmittel sind beispielsweise Alkohole, wie Methanol, Aethanol oder Isopropanol, Aether, wie Diäthyläther, Dioxan, Tetrahydrofuran, Glykolmonomethyläther oder Glykoldimethyläther, Dimethylformamid, Dimethylsulfoxid oder Acetonitril. Obwohl der Druck nicht kritisch ist und man die Umsetzung ohne weiteres bei erhöhtem Druck durchführen kann, arbeitet man aus Zweckmäsigkeitsgründen vorzugsweise bei Normaldruck. Die beiden Ausgangsstoffe werden vorzugsweise in äquimolaren Mengen eingesetzt.

Die Ylidenverbindungen der Formel II sind neu und ebenfalls Gegenstand vorliegender Erfindung. Sie können aus Ketosulfonamiden der allgemeinen Formel

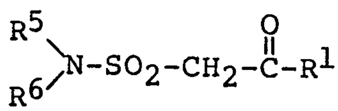

IV

worin $R^1$, $R^5$ und $R^6$ die oben angegebene Bedeutung besitzen,

durch Umsetzen mit einem Aldehyd der allgemeinen Formel

RCHO    V

worin R die oben angegebene Bedeutung besitzt,

hergestellt werden. Die Umsetzung erfolgt nach an sich bekannten Methoden unter Wasser-abspaltenden Bedingungen, beispielsweise durch Erhitzen zum Rückfluss in einem inerten organischen Lösungsmittel, wie einem aromatischen Kohlenwasserstoff, z.B. Benzol oder Toluol, und azeotropes Entfernen des gebildeten Wassers. Die Ketosulfonamide der Formel IV sind bekannt oder können in analoger Weise wie in

der Literatur beschrieben hergestellt werden.

Die Aldehyde der obigen Formel V sind bekannt oder können in analoger Weise zur Herstellung der bekannten Verbindungen erhalten werden.

Die Verbindungen der allgemeinen Formel I enthalten mindestens ein asymmetrisches Zentrum (4-Stellung) und können deshalb als optische Antipoden oder als Racemate vorliegen. Verbindungen der Formel I, die mehr als ein asymmetrisches Zentrum enthalten, können in verschiedenen diastereoisomeren Formen vorliegen. Die vorliegende Erfindung umfasst sämtliche möglichen Stereoisomeren von Verbindungen der allgemeinen Formel I und alle möglichen diastereoisomeren Mischungen und Racemate, sowie die Auftrennung dieser diastereoisomeren Mischungen, die nach an sich bekannten Methoden durchgeführt werden kann.

Die Verbindungen der Formel I besitzen eine ausgeprägte Calcium-antagonistische Wirkung und können deshalb als Arzneimittel verwendet werden, insbesondere für die Bekämpfung bzw. Verhütung von Angina pectoris, Ischämie, Bluthochdruck und Migräne.

Die Calcium-antagonistische Wirkung sowie die blutdrucksenkenden Eigenschaften der erfindungsgemässen Verbindungen können in den nachstehend beschriebenen Tests gezeigt werden:

A. $^{3}$H-Nifedipin-Bindungsbestimmungen:
Die Bestimmung wird an Homogenaten oder an partiell-gereinigten Membranen vom Kaninchen- oder Meerschweinchen-Herz durchgeführt. Die Reaktionsmischung (0,3 ml) besteht aus 0,2-0,8 mg Membranprotein, 1 nM $^{3}$H-Nifedipin (oder 0,25 nM $^{3}$H-Nitrendipin) und verschiedenen Konzentrationen der Prüfsubstanzen. Die Inkubation dauert 30 Minuten bei 25°C oder 37°C und wird durch Verdünnung mit dem Inkubationspuffer gestoppt; anschliessend erfolgt eine Filtration. Die filtergebundene Radioaktivität wird mit einem Szintillationszähler gemessen. Spezifische Bindung (d.h. rezeptorgebundene) wird als die Differenz zwischen total- und unspezifischgebundener Radioaktivität definiert. Die unspezifische Bindung wird in Gegenwart von einem Ueberschuss nichtradioaktivem Nifedipin (1µM) bestimmt.

Die Wirksamkeit (Potenz) einer Verbindung in diesem Test wird durch die $IC_{50}$- und % Maximale Hemmungs-Werte (% max. Inhibition) definiert. $IC_{50}$ ist die Substanzkonzentration (in Mol/l), die eine halb-maximale Inhibition der spezifischen $^{3}H$-Nifedipin (bzw. $^{3}H$-Nitrendipin)--Bindung verursacht. Die maximale Hemmung der spezifischen Bindung wird durch den % Maximale-Hemmungs-Wert angegeben; für die Referenzverbindung Nifedipin ist dieser Wert als 100% festgesetzt. Beide Parameter werden aus einer Konzentration-Bindung-Kurve extrapoliert.

B. <u>Hämodynamische Parameter am narkotisierten Hund:</u>
Die 4 wichtigsten Messparameter (mit resp. Messeinheiten) der hämodynamischen Versuche sind: (1) CBF: Coronary Blood Flow (in ml/min) - die Blutflussgeschwindigkeit durch die Koronararterien; (2) HR: Heart Rate (in Schläge/min) - die Herzfrequenz; (3) BP: Blood Pressure (in mm Hg) - der Blutdruck; und (4) dp/dt: Rate of increase in left ventricular pressure (in mm Hg/sec) - die Anstiegsgeschwindigkeit des linksventrikulären Druckes, als Mass für die Kontraktilitätskraft des Herzens. Die Werte werden als % maximale Aenderung vom Ausgangswert (Δ%) und Zeitdauer dieser Aenderung (t) pro verabreichter Dosis angegeben.

Dadurch bekommt man nicht nur ein Gesamtbild der Substanzwirkung, sondern auch eine Abschätzung über die potentielle Selektivität für einen bestimmten Teil des Kreislaufsystems im ganzen Organismus. Nach Verabreichung eines Anästhetikums, wird der Hund intubiert und künstlich

beatmet. Blut pH, $pCO_2$, $pO_2$ und Hämoglobin werden mit einem Blut-Gas-Analysator stündlich gemessen. Der Blutdruck (systolisch und diastolisch) wird mit einer Sonde in der Aorta abdominalis gemessen. Die Herzfrequenz wird mittels eines Tachometers erfasst, der vom Druckpuls ausgelöst wird. Für die anderen Messungen muss das Herz zuerst freigelegt werden, um eine Sonde in den linken Ventrikel (Herzkammer) für die Druckmessungen (dp/dt) einsetzen zu können. Der Coronarblutfluss wird mit einer Fluss-Sonde an der linken Coronararterie (descendens) gemessen.

Die in diesen Tests erhaltenen Resultate sind in der folgenden Tabelle zusammengefasst:

**Tabelle**

| Verbindung | A | | B | | | | |
|---|---|---|---|---|---|---|---|
| | $IC_{50}$ [M] | % max. Inhib. | CBF ml/min. t | HR Schläge/min. t | BP mmHg t | dp/dt mmHg/sec. t | Dosis mg/kg p.o. |
| A | $4,4 \cdot 10^{-9}$ | 100 | 50 (60') | -19 (40') | -55 (60') | -42 (60') | 0,03 |
| B | $9,5 \cdot 10^{-9}$ | 100 | 78 (40') | -11 (>30') | -30 (24') | - 2 (20') | 0,03 |
| C | $9,0 \cdot 10^{-9}$ | 100 | 108 (20') | -11 (>60') | -32 (>60') | -16 (>60') | 0,03 |

A = 5-(Dimethylsulfamoyl)-1,4-dihydro-2,6-dimethyl-4-(3-nitrophenyl)nicotinsäure-2-propoxyäthylester

B = 5-(Butylmethylsulfamoyl)-1,4-dihydro-2,6-dimethyl-4-(3-nitrophenyl)nicotinsäureisopropylester

C = 1,4-Dihydro-2,6-dimethyl-5-[(2-methoxyäthyl)methylsulfamoyl]-4-(3-nitrophenyl)nicotinsäureisopropylester

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verbindungen der Formel I mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man Verbindungen der allgemeinen Formel I bei der Bekämpfung bzw. Verhütung von Angina pectoris, Ischämie, Bluthochdruck und Migräne verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 bis 100 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

## Beispiel 1

Eine Lösung von 3,0 g (0,001 Mol) α-Acetyl-N,N-dimethyl-3-nitrostyrolsulfonamid und 1,43 g (0,01 Mol) 3-Aminocrotonsäureisopropylester in 30 ml Isopropanol wird 2 Stunden zum Rückfluss erhitzt, hierauf mit 1,16 g (0,005 Mol) DL-Campher-10-sulfonsäure versetzt und dann weitere 15 Minuten zum Rückfluss erhitzt. Nach dem Einengen unter vermindertem Druck wird der ölige Rückstand zwischen Wasser und Methylenchlorid verteilt, die organische Phase mit einer gesättigten wässrigen Lösung von Natriumchlorid gewaschen, über Natriumsulfat getrocknet und zur Trockene eingeengt. Das verbleibende gelbe Oel wird an 350 g Kieselgel mit Methylenchlorid/Essigester (9:1) als Eluiermittel chromatographiert. Die homogenen Fraktionen liefern ein Oel, welches durch Anreiben mit Aether kristallisiert. Nach Umkristallisation aus Methylenchlorid/Aether erhält man 3,2 g (75,5%) 5-(Dimethylsulfamoyl)-1,4-dihydro-2,6-dimethyl -4-(3-nitrophenyl)nicotinsäureisopropylester, Smp. 136-138°, als gelbliches Kristallpulver.

Das als Ausgangsmaterial verwendete α-Acetyl-N,N--dimethyl-3-nitrostyrolsulfonamid kann wie folgt hergestellt werden:

Eine Lösung von 20 g (0,16 Mol) N,N-Dimethylmethansulfonamid in 200 ml trockenem Tetrahydrofuran wird unter Argon tropfenweise zuerst bei 15-20° mit 100 ml (0,16 Mol) einer 1,6 molaren n-Butyllithium-Lösung in Hexan und dann bei 10-15° mit einer Lösung von 14,5 g (0,08 Mol) Essigsäure-4-nitrophenylester in 40 ml trockenem Tetrahydrofuran versetzt. Man lässt dann die Temperatur auf Raumtemperatur steigen (ca. 30 Minuten) und rührt 30 Minuten weiter. Nach dem Einengen unter vermindertem Druck wird der schwarze Rückstand zwischen 350 ml Eiswasser und 150 ml Aether verteilt. Die wässrige Phase wird mit 100 ml 2N Salzsäure angesäuert und mit Methylenchlorid extrahiert. Die über Natriumsulfat getrockneten Extrakte werden dann zur Trockene eingedampft. Das verbleibende Oel wird an 500 g Kieselgel mit Toluol/Essigester (4:1) als Eluiermittel chromatographiert. Die einheitlichen Fraktionen liefern 10,2 g (38%) N,N-Dimethyl-2-oxopropansulfonamid als Oel, welches ohne weitere Reinigung für die nächste Stufe eingesetzt werden kann. Das Oel kristallisiert langsam beim Stehenlassen. Für die Analyse wird ein Muster im Hochvakuum sublimiert, wobei man farblose Kristalle, Smp. 47-49°, erhält.

Eine Lösung von 4,1 g (0,025 Mol) N,N-Dimethyl-2-oxopropansulfonamid und 3,8 g (0,025 Mol) 3-Nitrobenzaldehyd in 25 ml Benzol wird mit 0,1 ml Piperidin und 0,3 ml Eisessig versetzt und hierauf 1 Stunde unter Wasserabscheiden zum Rückfluss erhitzt. Die Reaktionslösung wird dann unter vermindertem Druck zur Trockene eingeengt, der Rückstand in 20 ml Toluol gelöst, und die erhaltene Lösung wiederum zur

Trockene eingedampft. Das verbleibende Oel wird an 170 g Kieselgel mit Methylenchlorid als Eluiermittel chromatographiert. Die einheitlichen Fraktionen liefern 4,3 g (58%) α-Acetyl-N,N-dimethyl-3-nitrostyrolsulfonamid, Smp. 115-120°, als farbloses Kristallpulver.

## Beispiel 2

In analoger Weise wie in Beispiel 1 beschrieben wurden die folgenden Verbindungen erhalten:

- 5-(Dimethylsulfamoyl)-1,4-dihydro-2,6-dimethyl-4-(3-nitrophenyl)nicotinsäuremethylester, Smp. 166-169°, aus α-Acetyl-N,N-dimethyl-3-nitrostyrolsulfonamid und 3-Aminocrotonsäuremethylester;

- 5-(Dimethylsulfamoyl)-1,4-dihydro-2,6-dimethyl-4-(3-nitrophenyl)nicotinsäure-2-propoxyäthylester, Smp. 74-77°, aus α-Acetyl-N,N-dimethyl-3-nitrostyrolsulfonamid und 3-Aminocrotonsäure-2-propoxyäthylester;

- 5-(Butylmethylsulfamoyl)-1,4-dihydro-2,6-dimethyl-4-(3-nitrophenyl)nicotinsäureisopropylester, Smp. 80-83°, aus α-Acetyl-N-butyl-N-methyl-3-nitrostyrolsulfonamid und 3-Aminocrotonsäureisopropylester;

- 5-(Diäthylsulfamoyl)-1,4-dihydro-2,6-dimethyl-4-(3-nitrophenyl)nicotinsäureisopropylester, Smp. 133-136°, aus α-Acetyl-N,N-diäthyl-3-nitrostyrolsulfonamid und 3-Aminocrotonsäureisopropylester;

- 1,4-Dihydro-2,6-dimethyl-4-(3-nitrophenyl)-5-(1-pyrrolidinylsulfonyl)nicotinsäureisopropylester, Smp. 169-172°, aus 4-(3-Nitrophenyl)-3-(1-pyrrolidinylsulfonyl)-3-buten-2-on und 3-Aminocrotonsäureisopropylester und

- 1,4-Dihydro-2,6-dimethyl-5 -[(2-methoxyäthyl)methylsulfamoyl]-4 -(3-nitrophenyl)nicotinsäureisopropylester, Smp. 86-88°, aus α-Acetyl-N-(2-methoxyäthyl)-N-methyl-3--nitrostyrolsulfonamid und 3-Aminocrotonsäureisopropylester.

Die folgenden als Ausgangsmaterialien verwendeten 3-Nitrostyrolsulfonamide wurden ebenfalls in analoger Weise wie in Beispiel 1 beschrieben hergestellt:

- α-Acetyl-N-butyl-N-methyl-3-nitrostyrolsulfonamid in Form eines dicken Oels;

- α-Acetyl-N,N-diäthyl-3-nitrostyrolsulfonamid, Smp. 112-115°;

- 4-(3-Nitrophenyl)-3-(1-pyrrolidinylsulfonyl)-3-buten--2-on, Smp. 123-126° und

- α-Acetyl-N-(2-methoxyäthyl)-N-methyl -3-nitrostyrolsulfonamid, Smp. 86-88°.

## Beispiel 3

Gemäss der in Beispiel 1 beschriebenen Arbeitsweise wird eine Lösung von 3,12 g (0,01 Mol) α-Acetyl-N-isopropyl-3-nitrostyrolsulfonamid in 30 ml Isopropanol mit 1,87 g (0,01 Mol) 3-Aminocrotonsäure-2-propoxyäthylester und 1,16 g (0,005 Mol) DL-Campher-10-sulfonsäure versetzt. Nach Chromatographie an Kieselgel mit Methylenchlorid/-Essigester (4:1) als Eluiermittel wird das Rohprodukt aus Isopropanol umkristallisiert. Man erhält 1,0 g (21%) 5-(Isopropylsulfamoyl)-1,4-dihydro-2,6-dimethyl-4 -(3-nitrophenyl)nicotinsäure-2-propoxyäthylester, Smp. 155-158°, als farbloses Kristallpulver.

Das als Ausgangsmaterial verwendete α-Acetyl-N-isopropyl-3-nitrostyrolsulfonamid kann wie folgt hergestellt

werden:

Gemäss der in Beispiel 1 beschriebenen Arbeitsweise wird eine Lösung von 16,8 g (0,16 Mol) N-Isopropylmethansulfonamid in 200 ml trockenem Tetrahydrofuran mit 200 ml (0,32 Mol) einer 1,6 molaren n-Butyllithium-Lösung in Hexan und 14,5 g (0,08 Mol) Essigsäure-4-nitrophenylester in 40 ml trockenem Tetrahydrofuran versetzt. Das Rohprodukt wird an 1000 g Kieselgel mit Methylenchlorid/Essigester (9:1) als Eluiermittel chromatographiert. Die homogenen Fraktionen liefern 7,5 g N-Isopropyl-2-oxopropansulfonamid als Oel, welches ohne weitere Reinigung für die nächste Stufe eingesetzt werden kann.

In analoger Weise wie in Beispiel 1 beschrieben wird eine Lösung von 7,5 g (ca. 0,04 Mol) N-Isopropyl-2-oxopropansulfonamid und 6,0 g (0,04 Mol) 3-Nitrobenzaldehyd in 40 ml Benzol mit 0,16 ml Piperidin und 0,48 ml Eisessig versetzt. Das ölige Rohprodukt wird mit Aether angerieben, wobei Kristallisation eintritt. Man erhält 2,5 g α-Acetyl-N-isopropyl-3-nitrostyrolsulfonamid in Form von farblosen Kristallen, Smp. 153-157°.

## Beispiel 4

Gemäss der in Beispiel 1 beschriebenen Arbeitsweise wird eine Lösung von 3,67 g (0,01 Mol) α-(Cyclohexylcarbonyl)- -N,N-dimethyl-3-nitrostyrolsulfonamid in 30 ml Isopropanol mit 1,15 g (0,01 Mol) 3-Aminocrotonsäuremethylester und 1,16 g (0,005 Mol) DL-Campher-10-sulfonsäure versetzt. Nach Chromatographie an Kieselgel mit Methylenchlorid/Essigester (9:1) als Eluiermittel wird das Rohprodukt aus Acetonitril umkristallisiert. Man erhält 1,5 g (32%) 6-Cyclohexyl-5-(dimethylsulfamoyl)-1,4-dihydro-2-methyl-4-(3-nitrophenyl)nicotinsäuremethylester, Smp. 185-188°, als farbloses Kristallpulver.

Das als Ausgangsmaterial verwendete α-(Cyclohexylcarbonyl)-N,N-dimethyl-3-nitrostyrolsulfonamid wird in analoger Weise wie in Beispiel 1 beschrieben aus 5,8 g (0,025 Mol) 2-Cyclohexyl-N,N-dimethyl-2-oxoäthansulfonamid und 3,8 g (0,025 Mol) 3-Nitrobenzaldehyd hergestellt. Nach Chromatographie an Kieselgel mit Methylenchlorid als Eluiermittel erhält man 5,9 g (66%) α-(Cyclohexylcarbonyl)-N,N-dimethyl-3-nitrostyrolsulfonamid in Form von farblosen Kristallen, Smp. 115-118°.

<u>Beispiel 5</u>

Analog Beispiel 1 wird eine Lösung von 4,27 g (0,01 Mol) 6-(α-Acetyl-N-methyl-3 -nitrostyrolsulfonamido)-capronsäureäthylester in 30 ml Aethanol mit 1,15 g (0,01 Mol) 3-Aminocrotonsäuremethylester und 1,16 g (0,005 Mol) DL-Campher-10-sulfonsäure versetzt. Nach Chromatographie an Kieselgel mit Methylenchlorid/Essigester (9:1) als Eluiermittel wird das Rohprodukt aus Methylenchlorid/Aether umkristallisiert. Man erhält 3,4 g (65%) 5-{[5-(Aethoxycarbonyl)pentyl]methylsulfamoyl}-1,4 -dihydro-2,6-dimethyl--4-(3-nitrophenyl)nicotinsäuremethylester, Smp. 83-86°, als farbloses Kristallpulver.

Der als Ausgangsmaterial verwendete 6-(α-Acetyl-N--methyl-3 -nitrostyrolsulfonamido)capronsäureäthylester kann wie folgt hergestellt werden:

Eine Lösung von 72,7 g (0,4 Mol) 6-Aminocapronsäuremethylester-hydrochlorid in 2000 ml Methylenchlorid wird bei 20-25° zuerst mit 140 ml (1,0 Mol) Triäthylamin und dann mit einer Lösung von 38 ml (0,48 Mol) Methansulfochlorid in 160 ml Methylenchlorid versetzt. Die Mischung wird anschliessend 90 Minuten bei Raumtemperatur gerührt und hierauf mit 800 ml 0,5N Salzsäure versetzt. Die organische Phase wird mit gesättigter wässriger Natriumbicarbonat- sowie Natriumchlorid-Lösung gewaschen und über Natriumsulfat

getrocknet. Nach dem Einengen unter vermindertem Druck wird das verbleibende Oel in 400 ml Dimethylformamid gelöst, bei 15-20° mit einer Lösung von 0,48 Mol Natriummethylat (aus 11,0 g Natrium) in 100 ml Methanol versetzt und hierauf 30 Minuten bei Raumtemperatur gerührt. Das Methanol wird dann bei 50° unter vermindertem Druck abdestilliert, und die verbleibende Lösung bei 15-20° mit einer Lösung von 48 ml (0,8 Mol) Methyljodid in 100 ml Dimethylformamid versetzt. Das Reaktionsgemisch wird dann 15 Minuten bei Raumtemperatur weiter gerührt und hierauf unter vermindertem Druck zur Trockene eingeengt. Der Rückstand wird zwischen Wasser und Methylenchlorid verteilt, und die organische Phase mit einer gesättigten wässrigen Natriumchlorid-Lösung gewaschen, über Natriumsulfat getrocknet und zur Trockene eingedampft. Man erhält 90 g (95%) 6-[(Methylsulfonyl)-methylamino]capronsäuremethylester als Oel, welcher ohne weitere Reinigung für die nächste Stufe eingesetzt werden kann.

Eine Mischung von 90 g (0,38 Mol) 6-[(Methylsulfonyl)-methylamino]capronsäuremethylester und 67,5 g (0,76 Mol) 2-Amino-2-methyl-1-propanol wird 16 Stunden bei 135° gerührt. Das Reaktionsgemisch wird zwischen 500 ml Methylenchlorid und 250 ml 2N Salzsäure verteilt, und die organische Phase mit einer gesättigten wässrigen Natriumchlorid-Lösung gewaschen, über Natriumsulfat getrocknet und zur Trockene eingeengt. Das verbleibende Oel kristallisiert beim Anreiben mit Aether. Man erhält 85,2 g (76%) N-(2--Hydroxy-1,1-dimethyläthyl) -6-[(methylsulfonyl)methyl-amino]capronamid, Smp. 77-80°, als farbloses Kristallpulver. Für die Analyse wird ein Muster aus Essigester umkristallisiert, Smp. 84-87°.

Eine Lösung von 82,5 g (0,28 Mol) N-(2-Hydroxy-1,1--dimethyläthyl) -6-[(methylsulfonyl)methylamino]capronamid in 525 ml Methylenchlorid wird bei einer Temperatur zwischen -5 und 0° mit einer Lösung von 46,5 ml (0,64 Mol)

Thionylchlorid in 175 ml Methylenchlorid versetzt. Man lässt hierauf die Temperatur innerhalb von 15 Minuten auf 20° ansteigen. Die Lösung wird 45 Minuten bei Raumtemperatur weiter gerührt und danach wieder auf 0° abgekühlt. Das Reaktionsgemisch wird dann bei 0-5° mit einer Lösung von 220 g Kaliumcarbonat in 880 ml Wasser versetzt und hierauf 15 Minuten ohne Kühlung weiter gerührt. Die organische Phase wird dann mit einer gesättigten wässrigen Natriumchlorid-Lösung gewaschen, über Natriumsulfat getrocknet und zur Trockene eingeengt. Das verbleibende Oel wird an 1000 g Kieselgel mit Essigester/Aethanol (9:1) als Eluiermittel chromatographiert. Die homogenen Fraktionen liefern 62,2 g (80%) 4,5-Dihydro-4,4-dimethyl -2-{5-[(methylsulfonyl)-methylamino]pentyl}oxazol als gelbes Oel.

Gemäss der in Beispiel 1 beschriebenen Arbeitsweise wird eine Lösung von 22,1 g (0,08 Mol) 4,5-Dihydro-4,4--dimethyl -2-{5-[(methylsulfonyl)methylamino]pentyl}-oxazol in 100 ml trockenem Tetrahydrofuran mit 0,08 Mol Butyllithium und 7,25 g (0,04 Mol) Essigsäure-4-nitrophenylester versetzt. Nach dem Einengen unter vermindertem Druck wird der Rückstand zwischen Wasser und Aether verteilt, die wässrige Phase mit 80 ml 2N Salzsäure angesäuert und mit Methylenchlorid extrahiert. Die wässrige Lösung wird dann mit 2N Natronlauge auf pH 7 gestellt und wiederum mit Methylenchlorid extrahiert. Die über Natriumsulfat getrockneten Extrakte werden dann zur Trockene eingedampft. Der Rückstand wird an 250 g Kieselgel mit Essigester/-Aethanol (9:1) als Eluiermittel chromatographiert. Man erhält 7 g 4,5-Dihydro-4,4-dimethyl -2-{5-[(2-oxopropylsulfonyl)- methylamino]pentyl}oxazol als Oel, welches gemäss NMR-Daten noch ca. 30% des Ausgangsoxazols enthält.

Dieses Oel (7,0 g) wird in einer Mischung von 70 ml Aethanol und 2,5 ml Schwefelsäure gelöst, und die erhaltene Lösung 12 Stunden zum Rückfluss erhitzt. Das Reaktionsgemisch wird dann in 140 ml Eiswasser gegossen und mit Methylenchlorid extrahiert. Die organischen Extrakte werden vereinigt, mit gesättigter wässriger Natriumchlorid-Lösung gewaschen und über Natriumsulfat getrocknet. Nach dem Einengen unter vermindertem Druck wird der ölige Rückstand an 150 g Kieselgel mit Essigester/Aethanol (9:1) als Eluiermittel chromatographiert. Man erhält 6,5 g 6-[(2-Oxopropylsulfonyl)methylamino]capronsäureäthylester als Oel, welches gemäss NMR-Daten ca. 30% 6-[(Methylsulfonyl)methylamino]-capronsäureäthylester enthält.

Dieses Gemisch (6,5 g) wird in analoger Weise wie in Beispiel 1 beschrieben mit 3,0 g (0,02 Mol) 3-Nitrobenzaldehyd, 0,08 ml Piperidin und 0,24 ml Eisessig in 20 ml Benzol versetzt. Das ölige Rohprodukt wird an 250 g Kieselgel zuerst mit Methylenchlorid und dann mit Methylenchlorid/Essigester (9:1) als Eluiermittel chromatographiert. Die einheitlichen Fraktionen liefern ein Oel, welches durch Anreiben mit Aether kristallisiert. Man erhält 5,35 g 6-(α-Acetyl-N-methyl -3-nitrostyrolsulfonamido)capronsäureäthylester in Form von farblosen Kristallen, Smp. 60-63°.

## Beispiel 6

Eine Lösung von 3,42 g (0,01 Mol) α-Acetyl-N-(2--methoxyäthyl)-N-methyl-3-nitrostyrolsulfonamid und 1,87 g (0,01 Mol) 3-Aminocrotonsäure-2-propoxyäthylester in 15 ml Isopropanol wird 2 Stunden zum Rückfluss erhitzt, hierauf mit 1,0 g Amberlyst® 15 als Katalysator versetzt und dann weitere 30 Minuten zum Rückfluss erhitzt. Nach dem Einengen unter vermindertem Druck wird der ölige Rückstand in Methylenchlorid gelöst, der zurückgebliebene Katalysator abgenutscht, und das Filtrat mit einer gesättigten wässrigen Lösung von Natriumchlorid gewaschen, über Natriumsulfat getrocknet und zur Trockene eingeengt. Das verbleibende Oel wird an 300 g Kieselgel mit Methylenchlorid/Essigester (4:1) als Eluiermittel chromatographiert. Die einheitlichen

Fraktionen liefern ein Oel, welches durch Anreiben und Stehenlassen unter Hexan kristallisiert. Nach Umkristallisation aus Aether erhält man 2,4 g (47%) 1,4-Dihydro-2,6-dimethyl-5-[(2-methoxyäthyl)methylsulfamoyl] -4-(3-nitrophenyl)nicotinsäure-2-propoxyäthylester, Smp. 67-70°, als fast farbloses Kristallpulver.

Beispiel 7

In analoger Weise wie in Beispiel 6 beschrieben wurden die folgenden Verbindungen hergestellt:

- 4-(3-Chlorphenyl)-1,4-dihydro-2,6-dimethyl-5-[(2-methoxyäthyl)methylsulfamoyl]nictotinsäureisopropylester, Smp. 76-79°, aus α-Acetyl-3-chlor-N-(2-methoxyäthyl)-N-methylstyrolsulfonamid und 3-Aminocrotonsäureisopropylester;

- 4-(3-Chlorphenyl)-1,4-dihydro-2,6-dimethyl-5-[(2-methoxyäthyl)methylsulfamoyl]nicotinsäure -2-propoxyäthylester, Smp. 83-85°, aus α-Acetyl-3-chlor-N-(2-methoxyäthyl)-N-methylstyrolsulfonamid und 3-Aminocrotonsäure-2-propoxyäthylester;

- 4-(2,3-Dichlorphenyl)-1,4-dihydro-2,6-dimethyl-5-[(2-methoxyäthyl)methylsulfamoyl]nicotinsäureisopropylester, Smp. 134-136°, aus α-Acetyl-2,3-dichlor-N-(2-methoxyäthyl)-N-methylstyrolsulfonamid und 3-Aminocrotonsäureisopropylester;

- 1,4-Dihydro-5-dimethylsulfamoyl-2,6-dimethyl-4-(2-trifluormethylphenyl)nicotinsäuremethylester, Smp. 205-208°, aus α-Acetyl-N,N-dimethyl-2-trifluormethylstyrolsulfonamid und 3-Aminocrotonsäuremethylester;

- 4-(2-Chlorphenyl)-1,4-dihydro-2,6-dimethyl-5-[(2-methoxyäthyl)methylsulfamoyl]nicotinsäureisopropylester,

Smp. 107-110°, aus α-Acetyl-2-chlor-N-(2-methoxyäthyl)-N-methylstyrolsulfonamid und 3-Aminocrotonsäureisopropylester;

- 5-(Butylmethylsulfamoyl)-4-(2-chlorphenyl)-1,4-dihydro-2,6-dimethylnicotinsäureisopropylester, Smp. 98-101°, aus α-Acetyl-2-chlor-N-butyl-N-methylstyrolsulfonamid und 3-Aminocrotonsäureisopropylester;

- 5-(Butylmethylsulfamoyl)-1,4-dihydro-2,6-dimethyl-4-(3-nitrophenyl)nicotinsäurecyclopropylmethylester, Smp. 93-96°, aus α-Acetyl-N-butyl-N-methyl-3-nitrostyrolsulfonamid und 3-Aminocrotonsäurecyclopropylmethylester und

- 5-(Butylmethylsulfamoyl)-1,4-dihydro-2,6-dimethyl-4-(2-pyridyl)nicotinsäureisopropylester, Smp. 119-122°, aus N-Butyl-N-methyl-2-oxo-1-(2-pyridylmethylen)propansulfonamid und 3-Aminocrotonsäureisopropylester.

Die folgenden als Ausgangsmaterialien verwendeten Styrolsulfonamide wurden in analoger Weise wie in Beispiel 1 beschrieben hergestellt:

- α-Acetyl-3-chlor-N-(2-methoxyäthyl)-N-methylstyrolsulfonamid in Form eines dicken Oels;

- α-Acetyl-2,3-dichlor-N-(2-methoxyäthyl)-N-methylstyrolsulfonamid, Smp. 55-58°;

- α-Acetyl-N,N-dimethyl-2-trifluormethylstyrolsulfonamid in Form eines dicken Oels;

- α-Acetyl-2-chlor-N-(2-methoxyäthyl)-N-methylstyrolsulfonamid, Smp. 76-79°;

- α-Acetyl-2-chlor-N-butyl-N-methylstyrolsulfonamid, Smp. 65-67° und

- N-Butyl-N-methyl-2-oxo-1-(2-pyridylmethylen)propansulfonamid, Smp. 59-61°.

## Beispiel A

Herstellung von Tabletten folgender Zusammensetzung:

| | | |
|---|---|---|
| I. | 5-(Butylmethylsulfamoyl)-1,4-dihydro-2,6-dimethyl-4-(3-nitrophenyl)nicotinsäure-isopropylester, mikronisiert | 20,0 mg |
| | Milchzucker pulv. | 40,0 mg |
| | Maisstärke weiss | 24,9 mg |
| II. | Dioctylnatriumsulfosuccinat | 0,1 mg |
| | Maisstärke weiss | 5,0 mg |
| | Wasser | q.s. |
| III. | Maisstärke weiss | 6,0 mg |
| IV. | Talk | 3,0 mg |
| | Magnesiumstearat | 1,0 mg |
| | | 100,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Mischung wird zu Tabletten à 100 mg mit Bruchrille verpresst.

Beispiel B

Herstellung von Tabletten folgender Zusammensetzung:

| | | |
|---|---|---|
| I. | 5-(Butylmethylsulfamoyl)-1,4-dihydro-2,6-dimethyl-4-(3-nitrophenyl)nicotinsäure-isopropylester | 200,0 mg |
| | Milchzucker pulv. | 42,9 mg |
| | Maisstärke weiss | 50,0 mg |
| II. | Dioctylnatriumsulfosuccinat | 0,1 mg |
| | Maisstärke weiss | 20,0 mg |
| | Wasser | q.s. |
| III. | Maisstärke weiss | 30,0 mg |
| IV. | Talk | 3,5 mg |
| | Magnesiumstearat | 3,5 mg |
| | | 350,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die pressfertige Masse wird zu Tabletten à 350 mg mit Bruchrille verpresst.

## Beispiel C

Herstellung von Kapseln folgender Zusammensetzung:

| | | |
|---|---|---|
| I. | 5-(Butylmethylsulfamoyl)-1,4-dihydro-2,6-dimethyl-4-(3-nitrophenyl)nicotinsäure-isopropylester, mikronisiert | 20,0 mg |
| | Milchzucker pulv. | 48,0 mg |
| II. | Maisstärke | 5,0 mg |
| | Wasser | q.s. |
| III. | Milchzucker krist. | 50,0 mg |
| | Maisstärke | 15,0 mg |
| IV. | Talk | 10,0 mg |
| | Magnesiumstearat | 2,0 mg |
| | | 150,0 mg |

Die Stoffe der Phase I werden gesiebt und gemischt. Diese Mischung wird mit dem Maisstärkekleister II befeuchtet und geknetet. Die feuchte Masse wird granuliert, getrocknet und auf eine geeignete Korngrösse zugerüstet. Phase III wird beigemischt. Diese Mischung wird noch während kurzer Zeit mit Phase IV gemischt.

Die Kapselmischung wird zu je 150 mg in Kapseln Grösse 2 gefüllt.

## Beispiel D

Man stellt eine wässrige Tropfensuspension folgender Zusammensetzung her:

| | | 5 mg pro 1 ml |
|---|---|---|
| 5-(Butylmethylsulfamoyl)-1,4-dihydro-2,6-dimethyl-4-(3-nitrophenyl)nicotinsäure-isopropylester | | 0,05 g |
| Natriumbenzoat | | 0,035 g |
| Saccharin-Natrium | | 0,015 g |
| Acrylsäurepolymerisat | 0,1 - | 1,0 g |
| Saccharose | | 3,5 g |
| Citronensäure | | 0,025 g |
| Polyoxyäthylen-stearat | 0,002 - | 0,01 g |
| Natriumhydroxid | | q.s. |
| Aroma | | q.s. |
| Lebensmittelfarbstoff | | q.s. |
| Wasser entsalzt | ad | 10,0 ml |

## Beispiel E

Wenn man nach den in den Beispielen A-D beschriebenen Verfahren arbeitet, können aus den folgenden, ebenfalls bevorzugten Verbindungen Tabletten, Kapseln und Injektionspräparate hergestellt werden:

5-(Dimethylsulfamoyl)-1,4-dihydro-2,6-dimethyl -4-(3--nitrophenyl)nicotinsäure-2-propoxyäthylester und

1,4-Dihydro-2,6-dimethyl-5 -[(2-methoxyäthyl)methylsulfamoyl]-4 -(3-nitrophenyl)nicotinsäureisopropylester.

## Patentansprüche

1. Dihydropyridinderivate der allgemeinen Formel

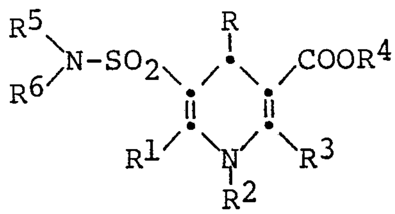

I

worin R Aryl oder einen heterocyclischen Rest mit bis zu drei Heteroatomen, wobei als Heteroatome Sauerstoff, Stickstoff oder Schwefel in Frage kommen, $R^1$ und $R^3$ je $C_1$-$C_4$-Alkyl oder $C_3$-$C_6$-Cycloalkyl, $R^2$ Wasserstoff oder $C_1$-$C_4$-Alkyl, $R^4$ $C_1$-$C_6$-Alkyl, $C_3$-$C_6$-Alkenyl, $C_3$-$C_6$-Alkinyl, $C_3$-$C_6$-Cycloalkyl, $C_3$-$C_6$-Cycloalkyl-$C_1$-$C_6$-alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_6$-Alkoxycarbonyl-$C_2$-$C_6$-alkyl, $C_1$-$C_4$-Alkanoyloxy-$C_1$-$C_6$-alkyl, $C_3$-$C_6$-Alkenyloxy-$C_1$-$C_6$-alkyl, $C_1$-$C_6$-Alkylthio-$C_1$-$C_6$-alkyl, Benzyloxy-$C_1$-$C_6$-alkyl, gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl oder gegebenenfalls durch Halogen, Cyan, Di-$C_1$-$C_6$-alkylamino, $C_1$-$C_6$-Alkoxy, $C_1$-$C_6$-Alkyl, Trifluormethyl oder Nitro substituiertes Phenyl-$C_1$-$C_6$-alkyl, $R^5$ Wasserstoff, $C_1$-$C_6$-Alkyl oder $C_3$-$C_6$-Cycloalkyl, $R^6$ $C_1$-$C_6$-Alkyl, $C_3$-$C_6$-Cycloalkyl, $C_3$-$C_6$-Cycloalkyl-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_6$-Alkylthio-$C_1$-$C_6$-alkyl, $C_1$-$C_6$-Alkoxycarbonyl-$C_2$-$C_6$-alkyl oder $R^5$ und $R^6$ zusammen die Gruppe $-CH_2CH_2-O-CH_2CH_2-$ oder eine $-(CH_2)_n$-Grup-

pe, worin n eine Zahl von 2 bis 7 bedeutet, bedeuten, in Form von Isomeren, Isomerengemischen, Racematen und optischen Antipoden.

2. Verbindungen gemäss Anspruch 1, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, $R^5$ $C_1$-$C_6$-Alkyl, $R^6$ $C_1$-$C_6$-Alkyl, $C_1$-$C_6$-Alkoxy-$C_1$-$C_6$-alkyl oder $C_1$-$C_6$-Alkoxycarbonyl-$C_2$-$C_6$-alkyl oder $R^5$ und $R^6$ zusammen eine $-(CH_2)_n-$Gruppe, worin n eine Zahl von 2 bis 7 bedeutet, und R Naphthyl, gegebenenfalls durch $C_1$-$C_6$-Alkyl, Halogen, Trifluormethyl oder Nitro monosubstituiertes oder durch Halogen bzw. Halogen und Nitro disubstituiertes Phenyl, Imidazolyl oder Pyridyl bedeuten.

3. Verbindungen gemäss Anspruch 1 oder 2, worin R Aryl, vorzugsweise durch $C_1$-$C_6$-Alkyl, Halogen, Trifluormethyl oder Nitro substituiertes Phenyl, $R^1$ Methyl, $R^2$ Wasserstoff und $R^3$ $C_1$-$C_4$-Alkyl, vorzugsweise Methyl, bedeuten.

4. Verbindungen gemäss einem der Ansprüche 1-3, worin $R^4$ $C_1$-$C_6$-Alkyl, Cyan-$C_2$-$C_6$-alkyl, Halogen-$C_2$-$C_6$-alkyl, Hydroxy-$C_2$-$C_6$-alkyl, ω,ω,ω-Trifluor-$C_1$-$C_6$-alkyl, $C_1$-$C_6$-Alkoxy-$C_1$-$C_6$-alkyl, $C_1$-$C_4$-Alkanoyloxy-$C_1$-$C_6$-alkyl, Benzyloxy-$C_1$-$C_6$-alkyl oder gegebenenfalls durch Halogen substituiertes Phenyl-$C_1$-$C_6$-alkyl, vorzugsweise $C_1$-$C_6$-Alkyl, ω,ω,ω-Trifluor-$C_1$-$C_6$-alkyl, $C_1$-$C_6$-Alkoxy-$C_1$-$C_6$-alkyl oder Phenyl-$C_1$-$C_6$-alkyl, ganz besonders bevorzugt Methyl, Isopropyl, 2,2,2-Trifluoräthyl, 2-Propoxyäthyl oder 1-Phenyläthyl, $R^5$ $C_1$-$C_6$-Alkyl, vorzugsweise Methyl, Aethyl oder Isopropyl, und $R^6$

$C_1$-$C_6$-Alkyl oder $C_1$-$C_6$-Alkoxy-$C_1$-$C_6$-alkyl, vorzugsweise Methyl, Aethyl, Isopropyl, Butyl oder Methoxyäthyl bedeuten.

5. Verbindungen gemäss einem der Ansprüche 1-4, worin R 3-Nitrophenyl, 2-Chlor-5-nitrophenyl oder 2,5-Dichlorphenyl, $R^1$ und $R^3$ je Methyl, $R^2$ Wasserstoff, $R^4$ Methyl, Isopropyl, 2,2,2-Trifluoräthyl, 2-Propoxyäthyl oder 1-Phenyläthyl, $R^5$ Methyl, Aethyl oder Isopropyl und $R^6$ Methyl, Aethyl, Isopropyl, Butyl oder Methoxyäthyl bedeuten.

6. Verbindungen gemäss einem der Ansprüche 1-5, worin R 3-Nitrophenyl, $R^1$ und $R^3$ je Methyl, $R^2$ Wasserstoff, $R^4$ Isopropyl oder 2-Propoxyäthyl, $R^5$ Methyl und $R^6$ Methyl, Butyl oder Methoxyäthyl bedeuten.

7. 5-[(Dimethylsulfamoyl)-1,4-dihydro-2,6-dimethyl-4-(3-nitrophenyl)nicotinsäure-2-propoxyäthylester.

8. 5-(Butylmethylsulfamoyl)-1,4-dihydro-2,6-dimethyl-4-(3-nitrophenyl)nicotinsäureisopropylester.

9. 1,4-Dihydro-2,6-dimethyl-5-[(2-methoxyäthyl)methylsulfamoyl]-4-(3-nitrophenyl)nicotinsäureisopropylester.

10. Verbindungen der allgemeinen Formel

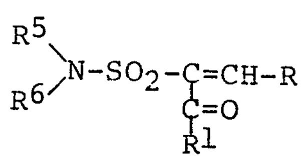

II

worin R Aryl oder einen heterocyclischen Rest mit bis zu drei Heteroatomen, wobei als Heteroatome Sauerstoff, Stickstoff oder Schwefel in Frage kommen, $R^1$ $C_1$-$C_4$-Alkyl oder $C_3$-$C_6$-Cycloalkyl, $R^5$ Wasserstoff, $C_1$-$C_6$-Alkyl oder $C_3$-$C_6$-Cycloalkyl, $R^6$ $C_1$-$C_6$-Alkyl, $C_3$-$C_6$-Cycloalkyl,

$C_3$-$C_6$-Cycloalkyl-$C_1$-$C_6$-alkyl, $C_1$-$C_6$-Alkoxy-$C_1$-$C_6$-alkyl, $C_1$-$C_6$-Alkoxy-$C_1$-$C_6$-alkoxy-$C_1$-$C_6$-alkyl, $C_1$-$C_6$-Alkylthio-$C_1$-$C_6$-alkyl, $C_1$-$C_6$-Alkoxycarbonyl-$C_2$-$C_6$-alkyl oder $R^5$ und $R^6$ zusammen die Gruppe $-CH_2CH_2-O-CH_2CH_2-$ oder eine $-(CH_2)_n$-Gruppe, worin n eine Zahl von 2 bis 7 bedeutet, bedeuten.

11. Dihydropyridinderivate gemäss einem der Ansprüche 1-9 zur Anwendung als therapeutische Wirkstoffe.

12. Dihydropyridinderivate gemäss einem der Ansprüche 1-9 zur Anwendung bei der Bekämpfung bzw. Verhütung von Angina pectoris, Ischämie, Bluthochdruck und Migräne.

13. Verfahren zur Herstellung einer Verbindung gemäss einem der Ansprüche 1-9, dadurch gekennzeichnet, dass man eine Ylidenverbindung der allgemeinen Formel

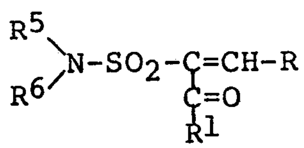

II

worin R, $R^1$, $R^5$ und $R^6$ die in Anspruch 1 angegebene Bedeutung besitzen,

mit einem Enamin der allgemeinen Formel

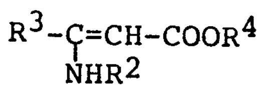

III

worin $R^2$, $R^3$ und $R^4$ die in Anspruch 1 angegebene Bedeutung besitzen,

umsetzt, und erwünschtenfalls, ein erhaltenes Isomerengemisch in die Isomeren auftrennt.

14. Arzneimittel, enthaltend ein Dihydropyridinderivat gemäss einem der Ansprüche 1-9 und ein therapeutisch inertes Excipiens.

15. Mittel zur Bekämpfung bzw. Verhütung von Angina pectoris, Ischämie, Bluthochdruck und Migräne, enthaltend ein Dihydropyridinderivat gemäss einem der Ansprüche 1-9 und ein therapeutisch inertes Excipiens.

16. Verwendung eines Dihydropyridinderivates gemäss einem der Ansprüche 1-9 bei der Bekämpfung bzw. Verhütung von Krankheiten.

17. Verwendung eines Dihydropyridinderivates gemäss einem der Ansprüche 1-9 bei der Bekämpfung bzw. Verhütung von Angina pectoris, Ischämie, Bluthochdruck und/oder Migräne.

18. Verwendung eines Dihydropyridinderivates gemäss einem der Ansprüche 1-9 zur Herstellung eines Arzneimittels gegen Angina pectoris, Ischämie, Bluthochdruck und/oder Migräne.

***

0212107

Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 86107778.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 616 995 (BAYER)<br>* Ansprüche 1,3,4 *<br>-- | 1,14, 18 | C 07 D 211/90<br>C 07 D 401/04<br>C 07 C 143/72<br>A 61 K 31/44 |
| A | DE - A1 - 2 524 277 (CIBA-GEIGY)<br>* Ansprüche 21,41 *<br>-- | 1,14 | |
| A | DE - A1 - 3 323 511 (HOECHST)<br>* Anspruch 3 *<br>-- | 1,10 | |
| A | EP - A1 - 0 134 934 (HOECHST)<br>* Anspruch 3 *<br>---- | 1,10 | |

RECHERCHIERTE SACHGEBIETE (Int. Cl.4)

C 07 D 211/00
C 07 C 143/00
C 07 D 401/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-15,18
Unvollständig recherchierte Patentansprüche: –
Nicht recherchierte Patentansprüche: 16,17
Grund für die Beschränkung der Recherche:

Verfahren zur therapeutischen Behandlung des menschlichen oder tierischen Körpers (Artikel 52 (4), EPÜ

| Recherchenort | Abschlußdatum der Recherche | Prüfer |
|---|---|---|
| WIEN | 05-09-1986 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 06.82